# EUROPÄISCHE PATENTSCHRIFT

(11) **EP 0 711 420 B1**
(45) Veröffentlichungstag und Bekanntmachung des Hinweises auf die Patenterteilung: **28.01.1998**
(21) Anmeldenummer: 94922819.1
(22) Anmeldetag: 15.07.1994
(51) Int. Cl.: G02B 6/30, G02B 6/36, H01L 21/306, H01L 21/302

(54) **VERFAHREN ZUM HERSTELLEN EINES SILIZIUM-HALBLEITERSUBSTRATS MIT INTEGRIERTEM WELLENLEITER UND DARAN ANGEKOPPELTER OPTISCHER FASER**
PROCESS OF MANUFACTURING A SILICON SEMICONDUCTOR SUBSTRATE WITH AN INTEGRATED WAVEGUIDE COUPLED TO AN OPTICAL FIBRE
PROCEDE DE FABRICATION D'UN SUBSTRAT SEMI-CONDUCTEUR EN SILICIUM AVEC UN GUIDE D'ONDES INTEGRE COUPLE A UNE FIBRE OPTIQUE

(30) Priorität: 27.07.1993 DE 4325955
(43) Veröffentlichungstag der Anmeldung: 15.05.1996
(73) Patentinhaber: SIEMENS AKTIENGESELLSCHAFT, 80333 München (DE)
(72) Erfinder: SPLETT, Armin, D-80689 München (DE)
(86) Internationale Anmeldenummer: DE9400867
(87) Internationale Veröffentlichungsnummer: WO9504296

(56) Entgegenhaltungen:
- EP-A- 0 361 153
- DE-A- 4 142 850
- PATENT ABSTRACTS OF JAPAN vol. 9, no. 56 (E-302) (1779) 12. März 1985 & JP,A,59 197 184 (NIPPON DENKI)

## Beschreibung

Die Erfindung bezieht sich auf ein Verfahren zum Herstellen eines Silizium-Halbleitersubstrats mit integriertem Wellenleiter und daran angekoppelter optischer Faser, bei dem der integrierte Wellenleiter durch Erzeugen einer Ausnehmung in einer Oberfläche des Silizium-Halbleitersubstrats mit einer frei zugänglichen Endfläche derart versehen wird, daß die Endfläche an der Ausnehmung gegenüber dem Ende einer in dieselbe Oberfläche des Silizium-Halbleitersubstrat anisotrop geätzten V-förmigen Nut liegt, und in die Nut die optische Faser bis zur frei zugänglichen Endfläche des integrierten Wellenleiters reichend eingelegt wird.

Aus einem Aufsatz von M.F. Grant, S. Day und R. Bellerby "Low-loss coupling of ribbon fibres to silica-on-silicon integrated optics using preferentially etched V-grooves" in "Integrated Photonics Research", (Summaries of papers presented at the Integrated Photonics Research Topical Meeting, 13. - 16. April 1992, New Orleans - Technical Digest Series, Vol. 10, Seiten 166 - 167) ist es bekannt, ein Halbleitersubstrat mit integriertem Wellenleiter und an diesem angekoppelter optischer Faser in der Weise herzustellen, daß in das Halbleitersubstrat zunächst eine V-förmige Nut durch anisotropes Ätzen eingebracht wird. Danach werden Wellenleiterschichten auf dem Halbleitersubstrat aufgebracht. Anschließend wird ein kanalartiger Wellenleiter dadurch gebildet, daß ein reaktives Ionen-Ätzen erfolgt. Das soweit vorbereitete Halbleitersubstrat wird anschließend durch Sägen mit einer Nut in der Weise versehen, daß eine senkrecht verlaufende Endfläche des integrierten Wellenleiters frei zugänglich geschaffen wird. Danach wird in die V-förmige Nut die optische Faser bis zur frei zugänglichen Endfläche des integrierten Wellenleiters reichend eingelegt. Auf diese Weise ist ein Silizium-Halbleitersubstrat mit integriertem Wellenleiter und angekoppelter optischer Faser gewonnen, bei dem die optische Faser zur frei zugänglichen Endfläche des integrierten Wellenleiters ausgerichtet ist, sofern der Sägeschnitt entsprechend genau ausgerichtet ausgeführt ist.

Ein weiteres Beispiel einer Methode zur Herstellung von Stirnflächen ist aus JP-A-59-197 184 bekannt.

Der Erfindung liegt die Aufgabe zugrunde, ein Verfahren zum Herstellen eines Silizium-Halbleitersubstrats mit integriertem Wellenleiter und daran angekoppelter optischer Faser vorzuschlagen, mit dem sich auf kostengünstige Weise unter sehr präziser Ausrichtung der optischen Faser zur frei zugänglichen Endfläche des integrierten Wellenleiters ein Silizium-Halbleitersubstrat herstellen läßt, das sich durch hohe optische Qualität der Ankopplungsstelle auszeichnet.

Zur Lösung dieser Aufgabe wird bei einem Verfahren der eingangs angegebenen Art erfindungsgemäß die Ausnehmung in dem Silizium-Halbleitersubstrat durch Heraustrennen eines Teilstücks des Halbleitersubstrats gebildet, indem das herauszutrennende Teilstück des Substrats so gewählt wird, daß sich das Endstück des integrierten Wellenleiters in das Teilstück erstreckt, durch anisotropes Ätzen das Teilstück so vom Substrat getrennt und damit freigelegt wird, daß das Teilstück nur an einem das Endstück des Wellenleiters umgebenden Bereich mit dem Substrat verbunden bleibt, eine quer zum Wellenleiter liegende V-förmige Kerbe, die die Dicke des Substratmaterials an dieser Stelle schwächt, in das Substrat geätzt wird und das anisotrope Ätzen von der der einen Oberfläche gegenüberliegenden Oberfläche des Substrats erfolgt; durch Druckausübung auf das herauszutrennende Teilstück erfolgt ein Abbrechen an der V-förmigen Kerbe unter Bildung der frei zugänglichen Endfläche des integrierten Wellenleiters als Bruchfläche.

Es ist zwar aus der DE 41 42 850 Al bekannt, in einem Halbleitersubstrat mit integriertem Wellenleiter im Bereich der Koppelstelle zwischen dem Wellenleiter und der optischen Faser durch Tiefenätzen eine Ausnehmung in Form einer Ätzgrube vorzusehen, jedoch handelt es sich bei dem Halbleitersubstrat um ein Substrat aus einem III-V-Halbleitermaterial, und das Halbleitersubstrat ist aus mehreren Schichten mit unter anderem einer ein selektives Naßätzen ermöglichenden Ätzschicht aufgebaut. Durch die Ätzgrube wird diese Ätzschicht freigelegt, so daß durch selektives Naßätzen im Bereich der Ätzgrube ein Teil der Ätzschicht herausgelöst werden kann. Das herausgeätzte Teil der Ätzschicht liegt unter einem Steg mit Lasche, der beim Tiefenätzen stehen gelassen wurde. Steg und Lasche sind damit freigelegt, so daß durch Ultraschall die Lasche unter Bildung einer dämpfungsarmen optischen Stoßkopplung abgespalten werden kann.

Der wesentliche Vorteil des erfindungsgemäßen Verfahrens gegenüber dem Verfahren nach dem eingangs behandelten Aufsatz von Grant u.a. besteht darin, daß durch das anisotrope Ätzen das herauszutrennende Stück und auch die V-förmige Kerbe in ihrer Lage zueinander und zu der V-förmigen Nut genau festlegbar sind, so daß eine sehr genaue Ausrichtung der optischen Faser zu der frei zugänglichen Endfläche des integrierten Wellenleiters möglich ist. Ein weiterer wesentlicher Vorteil besteht darin, daß durch die von der Bruchfläche gebildete frei zugängliche Endfläche des integrierten Wellenleiters eine Kopplungsfläche gebildet ist, die sich durch eine sehr hohe Oberflächenqualität auszeichnet, was zu einer weiteren Steigerung der optischen Qualität der Ankopplung führt. Im Vergleich zu dem aus der oben beschriebenen DE 41 42 850 Al entnehmbaren Verfahren ergibt sich der Vorteil, daß auf ein Halbleitersubstrat mit einem Mehrschichten-Aufbau verzichtet werden kann; außerdem ist nur ein anisotropes Ätzverfahren durchzuführen.

Es wird bei dem erfindungsgemäßen Verfahren als vorteilhaft angesehen, wenn auf der der Nut tragenden Oberfläche gegenüberliegenden Oberfläche des Halbleitersubstrats zum Heraustrennen des Stücks des Halbleitersubstrats eine Maske aufgebracht wird, die im Bereich der zu bildenden frei zugänglichen Endfläche des integrierten Wellenleiters einen für das anisotrop wirkende Ätzmedium zugänglichen Streifen mit Abmessungen aufweit, die die Bildung der V-förmigen Kerbe beim Ätzen ermöglicht. Der besondere Vorteil dieser Ausgestaltung des erfindungsgemäßen Verfahrens besteht darin, daß zum Heraustrennen des Stücks aus dem Halbleitersubstrat eine einzige Maske in einem fotolithografischen Prozeß auf der der Nut tragenden Oberfläche gegenüberliegenden Oberfläche des Halbleitersubstrats aufgebracht werden kann; dadurch gestaltet sich das gesamte Herstellungsverfahren kostengünstig. Ein zusätzlicher Vorteil wird darin gesehen, daß die Größe der Bruchfläche durch die Maske bzw. den Streifen festlegbar ist, indem der im Bereich des integrierten Wellenleiters für das Ätzmedium zugängliche Streifen entsprechend der Stärke des Halbleitersubstrats bemessen wird.

Bei einer weiteren vorteilhaften Ausführungsform des erfindungsgemäßen Verfahrens erfolgt die Druckausübung in der Weise, daß das Halbleitersubstrat von einer Flüssigkeit oder einem Gas angeströmt wird. Damit ist unter anderem die vorteilhafte Möglichkeit gegeben, Halbleitersubstrate noch im Wafer-Verbund nach dem erfindungsgemäßen Verfahren kostengünstig herzustellen.

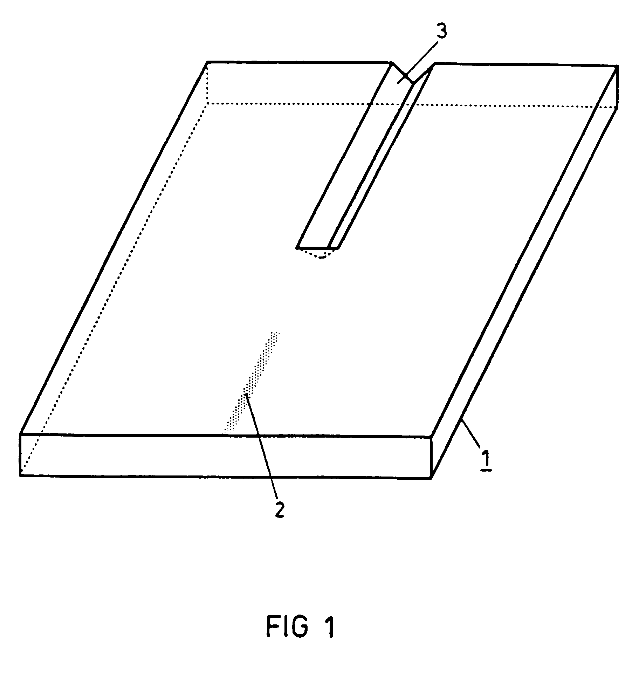

Zur Erläuterung der Erfindung ist in den Figuren 1 bis 4 eine Ausführungsform des erfindungsgemäßen Verfahrens anhand verschiedener gezeigter Fertigungszustände eines Halbleitersubstrats mit integriertem Wellenleiter und daran angekoppelter optischer Faser wiedergegeben.

In Figur 1 ist ein Halbleitersubstrat 1 dargestellt, das bereits mit einem integrierten optischen Wellenleiter 2 und einer V-förmigen Nut 3 versehen ist; die Herstellung des Halbleitersubstrats kann dabei in der Weise erfolgt sein, wie es eingangs bei der Erläuterung des bekannten Verfahrens beschrieben worden ist.

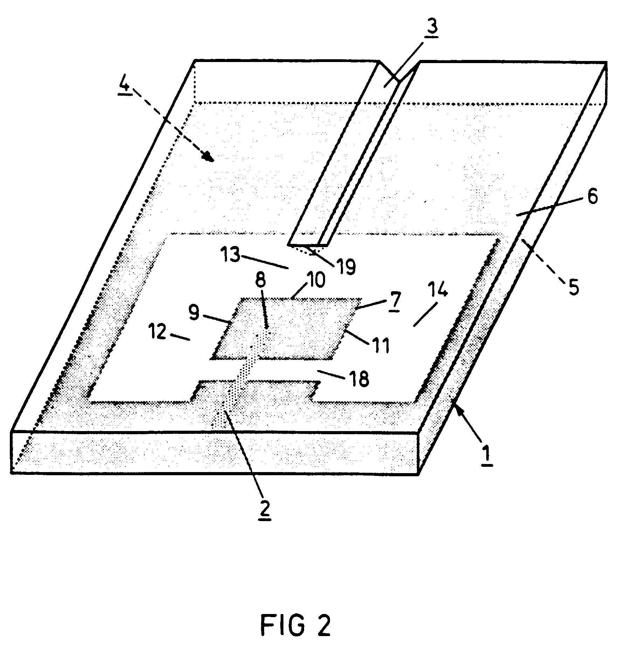

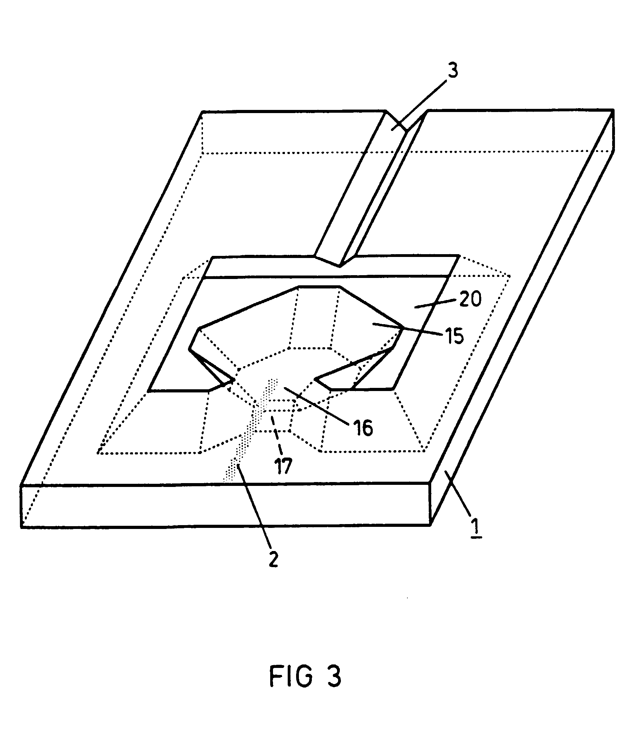

Das Halbleitersubstrat 1 in einem Fertigungszustand gemäß Figur 1 wird anschließend auf fotolithografischem Wege mit einer Maske 4 (vgl. Figur 2) auf einer Oberfläche 5 versehen, die der die V-förmige Nut 3 tragenden Oberfläche 6 gegenüberliegt. Die Maske 4 ist dabei so gestaltet, daß sie in einem Bereich 7 unterhalb des Endes 8 des integrierten Wellenleiters 2 eine Fläche abdeckt, die für das anisotrop wirkende Ätzmedium nicht zugänglich ist. An Rändern 9, 10 und 11 des Bereichs 7 grenzen relativ breit ausgeführte freie Flächen 12, 13 und 14 an, die im Hinblick auf die Wirkung des anisotrop wirkenden Ätzmittels so bemessen sind, daß beim Ätzen ein herauszutrennendes Stück 15 des Halbleitersubstrats 1 (vgl. Figur 3) bis auf einen den Wellenleiter 2 umgebenden Bereich 16 freigelegt wird. In diesem Bereich 16 wird nämlich nur die Bildung einer V-förmigen Kerbe 17 angestrebt, weshalb die Maske 4 im Bereich des integrierten Wellenleiters 2 einen für das anisotrop wirkende Ätzmedium zugänglichen Streifen 18 freiläßt. Dieser Streifen 18 ist im Hinblick auf die sich beim anisotropen Ätzen bildende V-förmige Kerbe 17 so gewählt, daß die Kerbe keinesfalls bis in den Bereich des integrierten Wellenleiters 2 reicht, sondern von der Oberfläche 6 betrachtet unterhalb des integrierten Wellenleiters 2 endet.

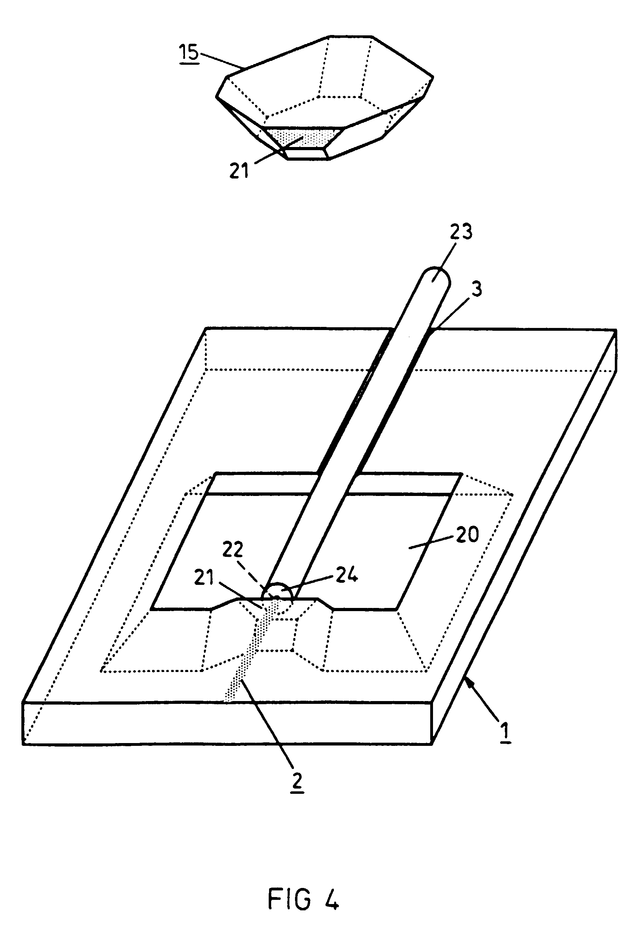

Das gemäß Figur 2 mit der beschriebenen Maske versehene Halbleitersubstrat 1 wird anschließend anisotrop geätzt, wodurch eine Konfiguration entsteht, wie sie in Figur 3 dargestellt ist. Deutlich läßt diese Figur erkennen, daß das herauszutrennende Stück 15 nur noch an der dem Ende 19 der V-förmigen Nut 3 gegenüberliegender Seite einer insoweit bereits teilweise gebildeten Ausnehmung 20 mit dem Halbleitersubstrat 1 in Verbindung steht. Wird das Halbleitersubstrat 1 in der Ausgestaltung nach Figur 3 anschließend von einer Flüssigkeit oder einem Gas angeströmt und dadurch ein Druck auf das insoweit freigelegte, herauszutrennende Stück 15 ausgeübt, dann bricht dieses im Bereich der V-förmigen Kerbe 17 ab. Dabei bildet sich eine senkrecht zur V-förmigen Nut 3 ausgerichtete Bruchfläche 21 (siehe Figur 4) von hoher optischer Qualität, in der der integrierte Wellenleiter 2 unter Bildung einer frei zugänglichen Endfläche 22 endet. Näheres bezüglich der optischen Qualität einer solchen Bruchfläche ist der DE-41 33 150 Al zu entnehmen.

Zur besseren Veranschaulichung ist in Figur 4 in der unteren Darstellung das Halbleitersubstrat 1 mit der nunmehr fertiggestellten Ausnehmung 20 und in der oberen Darstellung das herausgetrennte Stück 15 gezeigt. An dem Stück 15 ist die Bruchfläche 21 punktiert dargestellt, die in der unteren Darstellung der Figur 4 verdeckt ist.

Figur 4 läßt ferner erkennen, daß eine optische Faser 23 in die V-förmige Nut 3 eingelegt ist, und zwar so, daß sie mit ihrer Stirnseite 24 bis zur frei zugänglichen Endfläche 22 des integrierten Wellenleiters 2 bzw. bis Bruchfläche 21 reicht.

## Patentansprüche

1. Verfahren zum Herstellen eines Silizium-Halbleitersubstrats mit integriertem Wellenleiter und daran angekoppelter optischer Faser, bei dem
- der integrierte Wellenleiter durch Erzeugen einer Ausnehmung in einer Oberfläche des Silizium-Halbleitersubstrats mit einer frei zugänglichen Endfläche derart versehen wird, daß die Endfläche an der Ausnehmung gegenüber dem Ende einer in dieselbe Oberfläche des Silizium-Halbleitersubstrat anisotrop geätzten V-förmigen Nut liegt, und
- in die Nut die optische Faser bis zur frei zugänglichen Endfläche des integrierten Wellenleiters reichend eingelegt wird,
**dadurch gekennzeichnet,** daß
- die Ausnehmung (20) in dem Silizium-Halbleitersubstrat (1) durch Heraustrennen eines Teilstücks (15) des Halbleitersubstrats (1) gebildet wird, indem
- das herauszutrennende Teilstück (15) des Substrats (1) so gewählt wird, daß sich das Endstück des integrierten Wellenleiters (2) in das Teilstück (15) erstreckt,
- durch anisotropes Ätzen das Teilstück (15) so vom Substrat (1) getrennt und damit freigelegt wird, daß das Teilstück (15) nur an einem das Endstück des Wellenleiters (2) umgebenden Bereich (16) mit dem Substrat (1) verbunden bleibt,
- eine quer zum Wellenleiter (2) liegende V-förmige Kerbe (17), die die Dicke des Substratmaterials an dieser Stelle schwächt, in das Substrat (1) geätzt wird,
- das anisotrope Ätzen von der der einen Oberfläche gegenüberliegenden Oberfläche des Substrats (1) erfolgt, und
- durch Druckausübung auf das herauszutrennende Teilstück (15) ein Abbrechen an der V-förmigen Kerbe (17) unter Bildung der frei zugänglichen Endfläche (22) des integrierten Wellenleiters (2) als Bruchfläche (21) erfolgt.

2. Verfahren nach Anspruch 1,
**dadurch gekennzeichnet,** daß
- auf der der Nut (3) tragenden Oberfläche (6) gegenüberliegenden Oberfläche (5) des Silizium-Halbleitersubstras (1) zum Heraustrennen des Stücks (15) des Silizium-Halbleitersubstrats (1) eine Maske (4) aufgebracht wird, die
- im Bereich (7) der zu bildenden frei zugänglichen Endfläche (22) des integrierten Wellenleiters (2) einen für das anisotrop wirkende Ätzmedium zugänglichen Streifen (18) mit Abmessungen aufweist, die die Bildung der V-förmigen Kerbe (17) beim Ätzen ermöglichen.

3. Verfahren nach Anspruch 1 oder 2,
**dadurch gekennzeichnet,** daß
- die Druckausübung in der Weise erfolgt, daß das Halbleitersubstrat (1) von einer Flüssigkeit oder einem Gas angeströmt wird.

## Claims

1. Process for manufacturing a silicon semiconductor substrate having an integrated waveguide and an optical fibre connected to said waveguide, in which process
- by means of generating a recess in a surface of the silicon semiconductor substrate, the integrated waveguide is provided with a freely accessible end face, in such a way that the end face at the recess lies opposite the end of a V-shaped groove which has been anisotropically etched in the same surface of the silicon semiconductor substrate, and
- the optical fibre is placed in the groove in a manner such that it extends up to the freely accessible end face of the integrated waveguide,
characterised in that
- the recess (20) is formed in the silicon semiconductor substrate (1) by separating out a section (15) of the semiconductor substrate (1), with
- the section (15) of the substrate (1) that is to be separated out being chosen in such a way that the end piece of the integrated waveguide (2) extends into the section (15),
- by means of anisotropic etching, the section (15) being separated from the substrate (1) and thus exposed in such a way that the section (15) remains connected to the substrate (1) only at a region (16) surrounding the end piece of the waveguide (2),
- a V-shaped notch (17), which lies transversely with respect to the waveguide (2) and weakens the thickness of the substrate material at this place, being etched into the substrate (1),
- the anisotropic etching taking place from the surface of the substrate (1) that is opposite the one surface, and
- by means of the exertion of pressure on the section (15) to be separated out, a breaking off taking place at the V-shaped notch (17), forming the freely accessible end face (22) of the integrated waveguide (2) as a broken surface (21).

2. Process according to claim 1, characterised in that
- in order to separate out the piece (15) of the silicon semiconductor substrate (1), there is deposited on the surface (5) of the silicon semiconductor substrate (1) that is opposite the surface (6) carrying the groove (3), a mask (4) which,
- in the region (7) of the freely accessible end face (22) of the integrated waveguide (2) that is to be formed, has a strip (18) which is accessible for the anisotropically-acting etching medium and has dimensions which permit the formation of the V-shaped notch (17) during the etching.

3. Process according to claim 1 or 2,
characterised in that
- the exertion of pressure takes place in such a way that a liquid or a gas flows against the semiconductor substrate (1).

## Revendications

1. Procédé de fabrication d'un substrat semi-conducteur en silicium ayant un guide d'ondes intégré et une fibre optique qui y est couplée, qui consiste
- à munir le guide d'ondes intégré, en produisant un évidement dans une surface du substrat semi-conducteur en silicium, d'une surface d'extrémité accessible librement de façon que la surface d'extrémité sur l'évidement se trouve en face de l'extrémité d'une gorge en forme de V obtenue par attaque chimique anisotrope dans la même surface du substrat semi-conducteur en silicium et
- à insérer suffisamment la fibre optique dans la gorge jusqu'à la surface d'extrémité accessible librement du guide d'ondes intégré,
caractérisé en ce que
- l'évidement (20) est formé dans le substrat semi-conducteur (1) en silicium par enlèvement d'une partie (15) du substrat semi-conducteur (1) en
- choisissant la partie (15) du substrat (1) à enlever de manière que le tronçon d'extrémité du guide d'ondes (2) intégré s'étende dans la partie (15),
- en séparant par attaque chimique anisotrope la partie (15) du substrat (1) et ainsi en la dénudant de manière que la partie (15) ne reste reliée au substrat (1) que par une zone (16) entourant le tronçon d'extrémité du guide d'ondes (2),
- en ménageant par attaque chimique dans le substrat (1) une encoche (17) en forme de V qui est transversale au guide d'ondes (2) et qui affaiblit l'épaisseur du matériau du substrat en cet endroit,
- en effectuant l'attaque chimique anisotrope à partir de la surface du substrat (1) opposée à ladite une surface et,
- en effectuant par l'application d'une pression sur la partie (15) à séparer une rupture sur l'encoche (17) en forme de V avec formation de la surface (22) d'extrémité accessible librement du guide d'ondes (2) intégré sous la forme d'une surface (21) de rupture.

2. Procédé suivant la revendication 1,
caractérisé en ce qu'il consiste à déposer sur la surface (5) du substrat semi-conducteur (1) en silicium, opposée à la surface (6) portant la gorge (3) pour séparer la partie (15) du substrat semi-conducteur (1) en silicium, un masque (4) qui,
- comporte, dans la région (7) de la surface (22) d'extrémité accessible librement à former du guide d'ondes (2) intégré, une bande (18) accessible à l'agent d'attaque chimique agissant de manière anisotrope et ayant des dimensions qui permettent la formation de l'encoche (17) en forme de V lors de l'attaque chimique.

3. Procédé suivant la revendication 1 ou 2,
caractérisé en ce que
- l'application d'une pression s'effectue en faisant s'écouler un liquide ou un gaz sur le substrat semi-conducteur (1).
